# FASCICULE DE BREVET EUROPEEN

(11) **EP 0 525 236 B1**
(45) Date de publication et mention de la délivrance du brevet: **08.04.1998**
(21) Numéro de dépôt: 91112849.4
(22) Date de dépôt: 31.07.1991
(51) Int. Cl.: A61K 35/78

(54) **Procédé d'obtention d'un produit anti-diarrhéique à base de caroube**
Verfahren zur Herstellung eines Anti-Diarrhöe-Produktes auf Basis von Johannisbrot
Method for obtaining an anti-diarrhoea product based on carob

(43) Date de publication de la demande: 03.02.1993
(73) Titulaire: SOCIETE DES PRODUITS NESTLE S.A., 1800 Vevey (CH)
(72) Inventeur: Thomas, Remi, F-60390 Berneuil en Bray (FR)

(56) Documents cités:
- EP-A- 0 058 651
- EP-A- 0 214 317
- FR-A- 1 020 798

## Description

La présente invention est relative à un procédé d'obtention d'un produit anti-diarrhéique à base de caroube.

Les propriétés anti-diarrhéiques de la caroube sont bien connues. On connaît ainsi une composition contenant 60% à 80% en poids de farine de caroube torréfiée commercialisée sous la marque AROBON. Cette composition, qui donne de bons résultats dans le traitement des diarrhées, et sans effets secondaires, présente pourtant l'inconvénient de nécessiter des doses journalières de l'ordre de 20 g à 40 g, ce qui peut poser des problèmes d'administration, particulièrement chez les jeunes enfants.

On connaît ainsi, par le document EP 214317, un produit diététique anti-diarrhéique comprenant comme principe actif une farine de caroube.

Dans ce procédé, des cosses de caroube, après avoir été égrenées et concassées, sont moulues dans un broyeur et mises en présence d'eau. Les sucres contenus dans la caroube, ainsi que les tanins hydrosolubles à température ambiante, sont ainsi mis en solution. Après préparation de la phase liquide, on recueille un produit désucre.

Pour obtenir un produit débarrassé de ses micro-organismes, la caroube désucrée est ensuite mise en présence d'une nouvelle quantité d'eau et pasteurisée.

Après pasteurisation, la caroube subit une nouvelle mouture de façon à pouvoir être pulvérisée et séchée par atomisation avant de subir une mouture finale permettant d'obtenir une granulométrie autorisant sa mise en suspension dans un liquide.

Le produit obtenu, si il présente toutes caractéristiques d'un anti-diarrhéique efficace jointes à une grande facilité d'administration par rapport à l'art antérieur, présente pourtant plusieurs inconvénients de part son procédé d'obtention.

En premier lieu et comme expliqué dans le document EP 214317, l'effet anti-diarrhéique est obtenu grâce à la présence de tanins sous formes condensées, insolubles dans l'eau froide ou tiède et en particulier insolubles à la température du corps humain. Ainsi, ces tanins parviennent dans l'intestin sans être dégradés par l'acide gastrique ou inactivés par les protéines. Ils jouent ainsi un rôle dépuratif et antiseptique sans intervenir dans les processus physiologiques.

Il est donc primordial, pour obtenir un produit antidiarrhéique efficace, d'éviter une mise en solution des tanins présents dans la caroube désucrée. Or, cette mise en solution commence à des températures voisines de 90°C et est considérée comme complète à des températures de l'ordre de 120°C.

Ceci limite donc le traitement de pasteurisation qui est ainsi réalisé aux alentours de 95°C.

C'est ainsi que le produit final obtenu présente au moins 20% en poids de tanins natifs (exprimés en polyphénols totaux) avec un rapport pondéral tanins solubles sur tanins insolubles inférieur à 0,37, pour une mesure de solubilité à 37°C.

En second lieu, le procédé de séchage n'est pas économique puisqu'il suit une addition d'eau massive nécessaire à la pasteurisation, le produit avant séchage présentant une teneur en matières sèches comprise entre 20% et 25% en poids.

La présente invention a donc pour but de proposer un procédé d'obtention d'un produit anti-diarrhéique du type décrit dans EP 214317 dans lequel la pasteurisation est réalisée à de beaucoup plus hautes températures sans pour autant qu'apparaisse une mise en solution des tanins en permettant par ailleurs de réaliser les opérations de pasteurisation et de séchage en une seule étape.

L'utilisation de vapeur surchauffée pour pasteuriser des produits alimentaires est connue depuis fort longtemps, par exemple par le document FR 1.020.798.

Par ailleurs, on connaît par le document EP 58651 un procédé et un dispositif pour traiter des aliments pour animaux par vapeur surchauffée.

Grâce à ce procédé, et à ce dispositif, de traitement à la vapeur surchauffée, il est possible de sécher le produit à traiter dans des proportions fonction des paramètres de traitement que constituent la pression, la température de la vapeur surchauffée et le temps de traitement.

Or il a été trouvé qu'il est possible d'utiliser un traitement à la vapeur surchauffée, sur de la caroube désucrée, à des températures nettement supérieures à 120°C sans que pour autant apparaisse une solubilisation des tanins. Il est ainsi possible en une seule étape de pasteuriser et sécher la caroube désucrée.

La présente invention a ainsi pour objet un procédé d'obtention d'un produit anti-diarrhéique à base de caroube dans lequel les cosses de caroube, après avoir été égrenées et moulues, sont désucrées en milieu aqueux, la phase aqueuse étant ensuite séparée de la phase solide, le résidu solide obtenu étant ensuite simultanément séché et pasteurisé par traitement à la vapeur surchauffée.

D'autres caractéristiques et avantages ressortiront à la suite de la description qui va suivre.

La première étape du procédé consiste à préparer la caroube en vue de sa pasteurisation et de son séchage antérieur. Pour cela, il est possible d'opérer selon la manière décrite dans EP 214317.

Des cosses de caroube, après avoir été égrenées et concassées en morceaux de 1 à 2 cm, subissent une mouture dans un broyeur et une extraction des sucres. Ces deux opérations peuvent être séparées, la mouture étant réalisée en premier, l'extraction des sucres étant réalisée ensuite par mise en suspension de la caroube dans une certaine quantité d'eau. Préférablement, l'extraction des sucres peut être réalisée par immersion d'une partie en poids de caroube et 11 parties en poids d'eau, pendant 30 minutes. Il est aussi possible de réaliser le broyage dans un broyeur colloïdal ce qui permet de désucrer simultanément la caroube.

L'eau est ensuite partiellement extraite de la caroube désucrée, par exemple par essorage ou pressage.

Après cette opération d'extraction de l'eau, le produit se présente sous forme d'agrégats. Afin de faciliter les opérations de dosage et de séchage, il peut être utile d'émietter ces agrégats, par exemple grâce à un broyeur à marteaux. Il est ainsi obtenu un produit qui s'écoule très bien en ne se prenant pas en masse et qu'il faut désormais sécher et pasteuriser afin d'obtenir un produit final.

Ces opérations de pasteurisation et de séchage sont réalisées simultanément par traitement à la vapeur surchauffée.

Pour la mise en oeuvre du traitement à la vapeur surchauffée, il est possible d'utiliser un dispositif tel que décrit dans EP 58651 déjà mentionné.

La vapeur surchauffée est obtenue par chauffage de vapeur à une température supérieure à sa température de saturation.

Le traitement peut se faire à pression atmosphérique ou sous pression. Dans ce cas la vapeur est obtenue par surchauffe de vapeur saturée maintenue sous pression.

La vapeur surchauffée tend toujours à revenir à l'état de vapeur saturante en cédant sa chaleur sensible. Cette chaleur sensible peut ainsi être utilisée pour évaporer de l'eau et donc pour déshydrater un produit.

Dans ce document EP 58651, des tubes verticaux sont reliés entre eux par des tubes en U. Ces tubes verticaux comportent une double enveloppe alimentée en vapeur pour surchauffer la vapeur circulant à l'intérieur desdits tubes.

En amont du premier tube, un système d'alimentation composé d'une trémie, d'une première écluse et d'un dispositif d'injection de vapeur, par exemple un ventilateur, permet d'introduire et disperser le produit dans la vapeur surchauffée.

En aval du dernier tube, un cyclone sépare le produit traité de la vapeur. La vapeur est recyclée et surchauffée dans un échangeur de chaleur avant d'être réintroduite par un ventilateur dans le circuit tubulaire. Le produit traité est extrait du cyclone par une deuxième écluse. Les écluses assurent par ailleurs le maintien de la pression dans le circuit tubulaire de traitement. Un système de vannes permet la régulation de la pression à l'intérieur du circuit de traitement, par injection ou extraction de vapeur.

Dans ce type de dispositif, le produit à traiter est introduit sous pression par la première écluse. Ce produit est dispersé dans le flux de vapeur surchauffée qui transporte alors le produit dans le circuit tubulaire.

Lors du traitement à la vapeur surchauffée, le produit passe, dans une première étape, de la température ambiante à la température de saturation puis est séché. La température de saturation dépend, en vapeur surchauffée, de la pression.

Il était dans un premier temps fondamental de prouver que le traitement à la vapeur surchauffée n'entraînait aucune mise en solution des tanins présents dans la caroube désucrée. Pour cela, la caroube désucrée a été traitée à la vapeur surchauffée à trois pressions, et donc à trois températures de saturation différentes.

La mesure du niveau de solubilisation des tanins est réalisée de la façon suivante : 5 g du produit final, obtenu après traitement à la vapeur surchauffée, sont mis en suspension dans 100 ml d'eau. La suspension est agitée et la coloration est comparée à la coloration d'une suspension aqueuse obtenue à partir de la caroube désucrée non traitée à la vapeur surchauffée (témoin).

Les résultats sont résumés dans le tableau ci-dessous où le temps de traitement est indiqué sous la forme d'une limite supérieure, les particules ne se déplacent pas toutes a la même vitesse dans l'installation.

| Conditions de traitement | Essai A | Essai B | Essai C |
|---|---|---|---|
| Température de la vapeur (°C) | 153 | 180 | 198 |
| Température de saturation (°C) | 102 | 111 | 134 |
| Pression relative de la vapeur (bar) | 0,1 | 0,5 | 2 |
| Temps de traitement | 18-20 s | 19-50 s | 19-50 s |
| Coloration | absence | absence | absence |

On voit ainsi qu'au cours du traitement à la vapeur surchauffée, il ne se produit pas de solubilisation des tanins malgré des températures nettement supérieures à 120°C. Des mesures réalisées par spectrométrie ont confirmé l'absence de mise en solution des tanins.

Différents essais ont été réalisés en faisant varier :
- le débit du produit dans l'installation,
- le taux de matières sèches initial du produit,
- la pression (et donc la température de saturation),
- la température sèche de la vapeur introduite mesurée au ventilateur d'entrée.

Durant ces essais, le temps de traitement du produit était compris entre 15 et 60 secondes et la vitesse d'injection de la vapeur était comprise entre 26 et 28 m/s.

Il a par ailleurs été mesuré la température sèche de la vapeur dans le premier tube et dans le dernier tube de l'installation. En effet, au cours du traitement cette température évolue. Plus particulièrement, la vapeur cédant une grande partie de sa chaleur sensible dans le premier tube, sa température baisse jusgu'à pouvoir atteindre la température de saturation. Ceci est à éviter car cela peut entraîner une réduction de la vitesse d'écoulement de la vapeur et bloquer l'installation par accumulation de produit non transporté. La température de la vapeur remonte ensuite dans l'installation grâce aux doubles enveloppes des tubes.

Il a été enfin mesuré la teneur en matières sèches du produit final et la quantité d'eau évaporée par heure (en kg/h).

Les résultats des essais sont résumés dans le tableau récapitulatif ci-dessous, la pression de vapeur étant indiquée en valeur relative par rapport à la pression atmosphérique.

| Tableau récapitulatif des essais | | | | | | | | |
|---|---|---|---|---|---|---|---|---|
| Essai | 1 | 2 | 3 | 4 | 5 | 6 | 7 | 8 |
| Débit du produit (kg/h) | 10 | 10 | 10 | 15 | 20 | 30 | 40 | 60 |
| Matière sèche du produit (%) | 30,6 | 30,6 | 31,6 | 31,6 | 31,2 | 31,2 | 31,2 | 35,7 |
| Pression de vapeur (bar) | 0,5 | 2,0 | 0,5 | 0,5 | 0,5 | 0,5 | 0,5 | 0,5 |
| Température de saturation de la vapeur (°C) | 111 | 134 | 111 | 111 | 111 | 111 | 111 | 111 |
| Température de la vapeur au ventilateur (°C) | 180 | 198 | 195 | 192 | 198 | 198 | 197 | 200 |
| Matière sèche finale (%) | 96,8 | 89,8 | 97,3 | 97,1 | 96,1 | 94,6 | 91,7 | 87,6 |
| Eau évaporée (kg/h) | 6,84 | 6,50 | 6,75 | 10,12 | 13,51 | 20,11 | 26,39 | 35,56 |

On a noté, comme déjà dit ci-dessus, que la température de la vapeur ne provoque pas de solubilisation des tanins, de même, il n'apparaît pas de trace de brûlure sur le produit.

Le critère fondamental à prendre en compte pour le choix de la température de la vapeur, en ce qui concerne l'efficacité du séchage, est l'écart entre la température de la vapeur injectée dans l'installation mesurée au ventilateur d'entrée et la température de saturation, elle-même déterminée par la pression de vapeur. A température de vapeur constante, une augmentation de pression réduit le rendement évaporatoire.

Ceci est parfaitement illustré par le tableau ci-dessous qui montre l'influence de cet écart entre ces deux températures sur le taux de matières sèches final du produit.

| Influence de l'écart entre la température de la vapeur introduite et sa température de saturation | | | | | | | | |
|---|---|---|---|---|---|---|---|---|
| Essai | 1 | 2 | 3 | 4 | 5 | 6 | 7 | 8 |
| Débit du produit (kg/h) | 10 | 10 | 10 | 15 | 20 | 30 | 40 | 60 |
| Matière sèche du produit (%) | 30,6 | 30,6 | 31,6 | 31,6 | 31,2 | 31,2 | 31,2 | 35,7 |
| Pression de vapeur (bar) | 0,5 | 2,0 | 0,5 | 0,5 | 0,5 | 0,5 | 0,5 | 0,5 |
| Température de saturation de la vapeur (°C) | 111 | 134 | 111 | 111 | 111 | 111 | 111 | 111 |
| Température de la vapeur au ventilateur (°C) | 180 | 198 | 195 | 192 | 198 | 198 | 197 | 200 |
| Ecart de température (°C) | 69 | 64 | 84 | 81 | 87 | 87 | 86 | 89 |
| Matière sèche finale (%) | 96,8 | 89,8 | 97,3 | 97,1 | 96,1 | 94,6 | 91,7 | 87,6 |
| Eau évaporée (kg/h) | 6,84 | 6,50 | 6,75 | 10,12 | 13,51 | 20,11 | 26,39 | 35,56 |

On voit ainsi, plus particulièrement aux exemples 1,2,3 que, pour des quantités d'eau évaporées par heure très voisines, le débit d'introduction de la caroube désucrée étant le même, le taux de matières sèches final est directement lié à cet écart de température.

Le tableau ci-dessous illustre par ailleurs l'influence de la quantité d'eau à évaporer sur la baisse de température de la vapeur dans le premier tube et sur le taux de matières sèches final, tous les essais ayant été réalisés à une pression relative de vapeur de 0,5 bar.

La quantité d'eau à évaporer, c'est-à-dire la quantité d'eau introduite dans l'installation, est proportionnelle au débit et inversement proportionnelle au taux de matières sèches initial.

| Influence de la quantité à évaporer sur la baisse de température dans le premier tube et sur le taux de matière sèche finale | | | | | | | |
|---|---|---|---|---|---|---|---|
| Essai | 1 | 3 | 4 | 5 | 6 | 7 | 8 |
| Débit du produit (kg/h) | 10 | 10 | 15 | 20 | 30 | 40 | 60 |
| Matière sèche du produit (%) | 30,6 | 31,6 | 31,6 | 31,2 | 31,2 | 31,2 | 35,7 |
| Pression de vapeur (bar) | 0,5 | 0,5 | 0,5 | 0,5 | 0,5 | 0,5 | 0,5 |
| Température de la vapeur au ventilateur (°C) | 180 | 195 | 192 | 198 | 198 | 197 | 200 |
| Température de la vapeur dans le tube 1 (°C) | 149 | 157 | 145 | 148 | 136 | 126 | 121 |
| Ecart de température (°C) | 31 | 38 | 47 | 50 | 62 | 71 | 79 |
| Matière sèche finale (%) | 96,8 | 97,3 | 97,1 | 96,1 | 94,6 | 91,7 | 87,6 |
| Eau évaporée (kg/h) | 6,84 | 6,75 | 10,12 | 13,51 | 20,11 | 26,39 | 35,56 |

On voit clairement que plus la quantité d'eau à évaporer augmente, plus la température dans le premier tube baisse et plus le taux de matières sèches dans le produit final baisse alors même que la quantité d'eau évaporée par heure augmente.

Pour obtenir un produit final ayant un taux de matières sèches au moins égal à 90% en poids, il est donc nécessaire d'avoir une vapeur injectée à une température supérieure d'au moins 60°C par rapport à sa température de saturation et préférablement supérieure d'au moins 70°C.

Les conditions préférentielles de traitement à la vapeur surchauffée ont finalement été choisies comme suit :
- - température de la vapeur surchauffée: : 200°C
- - pression de vapeur (relative): : 0,5 bar
- - vitesse d'injection de la vapeur: : 27 m/s
- - temps de séjour du produit: : 20 à 60 s
- - débit d'entrée du produit: : 30 kg/h

En traitant la caroube désucrée avec ces paramètres il a été obtenu un produit final présentant un taux de matières sèches de 95% en poids présentant 23,2%, en poids de matières sèches, de tanins exprimés en polyphénols totaux, répartis en 0,8% de tanins solubles et 22,4% de tanins insolubles soit un rapport tanins solubles/tanins insolubles inférieur à 0,04.

Enfin, la pasteurisation obtenue est très efficace comme le montre le tableau ci-dessous, la pasteurisation étant par ailleurs d'autant plus efficace que la pression de vapeur est plus élevée.

| Germes | Produit avant traitement | Produit après traitement |
|---|---|---|
| Germes mésophiles revivifiables / g | 150 000 | < 3 000 * |
| Coliformes / g | > 3 000 | < 10 |
| Coliformes fécaux / g | > 10 | absence |
| Staphyloccocus aureus | absence | absence |
| Clostridium sulfito réducteurs | absence | absence |
| Levures / g | 4 300 | absence |
| Moisissures / g | 160 | absence |

| | | |
|---|---|---|
| * Limite de détection de l'analyse | | |

Le traitement selon l'invention permet donc effectivement de décontaminer très efficacement le produit, la réduction des germes étant supérieure à un facteur 1000.

## Revendications

1. Procédé d'obtention d'un produit anti-diarrhéique à base de caroube dans lequel les cosses de caroube, après avoir été égrenées et moulues, sont désucrées en milieu aqueux, la phase aqueuse étant ensuite séparée de la phase solide, le résidu solide obtenu étant ensuite simultanément séché et pasteurisé par traitement à la vapeur surchauffée.

2. Procédé selon la revendication 1 dans lequel la vapeur surchauffée dépasse d'au moins 60°C sa température de saturation.

3. Procédé selon la revendication 2 dans lequel le résidu solide obtenu est traité à la vapeur surchauffée pendant moins d'une minute.

## Claims

1. Process for obtaining an anti-diarrhoeic product based on carob, wherein carob pods are desugared in an aqueous medium, after having been deseeded and ground, the aqueous phase being then separated from the solid phase, the solid residue obtained being then simultaneously dried and pasteurized by treatment with superheated steam.

2. Process according to claim 1, wherein the superheated steam exceeds its saturation temperature by at least 60°C.

3. Process according to claim 2, wherein the solid residue obtained is treated with superheated steam for less than one minute.

## Patentansprüche

1. Verfahren zur Gewinnung eines anti-diarrhoischen Produkts auf der Basis von Johannisbrot, bei dem man Johannisbrothülsen, nachdem man sie entkernt und gemahlen hat, in einem wäßrigen Milieu entzuckert, die dabei erhaltene wäßrige Phase von der festen Phase abtrennt, und den auf diese Weise erhaltenen festen Rückstand gleichzeitig durch eine Behandlung mit überhitztem Dampf trocknet und pasteurisiert.

2. Verfahren nach Anspruch 1, bei dem der überhitzte Dampf die Sättigungstemperatur um wenigstens 60°C überschreitet.

3. Verfahren nach Anspruch 2, bei dem der erhaltene feste Rückstand mit dem überhitzten Dampf für weniger als eine Minute behandelt wird.
